# EUROPEAN PATENT SPECIFICATION

(11) **EP 2 558 162 B1**
(45) Date of publication and mention of the grant of the patent: **10.06.2020**
(21) Application number: 10720260.8
(22) Date of filing: 15.04.2010
(51) Int. Cl.: A61N 5/10, G01R 33/48

(54) **RADIOTHERAPY AND IMAGING APPARATUS**
STRAHLENTHERAPIE UND ABBILDUNGSVORRICHTUNG
APPAREIL DE RADIOTHÉRAPIE ET D'IMAGERIE

(43) Date of publication of application: 20.02.2013
(73) Proprietor: Elekta AB (PUBL), 103 93 Stockholm (SE)
(72) Inventor: LAGENDIJK, Jan, NL-3584 CX Utrecht (NL); RAAYMAKERS, Bas, NL-3584 CX Utrecht (NL)
(74) Representative: Haseltine Lake Kempner LLP
(86) International application number: PCT/EP2010/002325
(87) International publication number: WO 2011/127947

(56) References cited:
- EP-A1- 1 741 469
- WO-A1-00/07669
- WO-A1-2006/097274
- WO-A2-03/008986
- WO-A2-2005/081842
- US-A- 5 206 893
- US-A1- 2007 076 846
- US-A1- 2007 237 290
- US-A1- 2007 244 386

## Description

### FIELD OF THE INVENTION

The present invention relates to combined radiotherapy and imaging systems.

### BACKGROUND ART

One of the biggest problems in the field of radiotherapy at the moment is compensating for the motion of the target area, due to for example the breathing of the patient. A new method that may help with this is the combination of magnetic resonance imaging (MRI) with simultaneous radiation therapy. MRI allows three-dimensional imaging of the target region whilst simultaneously treating the patient with radiation. This helps to reduce the errors in beam position, which therefore reduces the amount of healthy tissue which is irradiated.

When using MRI, imaging is performed by applying an RF field in the presence of a magnetic field to a target region within a patient so that it reorients the spin of the nuclei of the atoms. A "readout" can be made by removing the RF field, and watching how long it takes these nuclei to relax back to their previous state. This information is then processed to produce an image. Images are generally taken in slices through the patient, so to build up a full three-dimensional picture a number of slice images have to be taken and combined together. The position and orientation of these slices in the patient are defined by a magnetic gradient field, generated by a set of gradient coils. This process is well known in the art.

The process of acquiring multiple slices is repetitive and takes an extended period of time, however, during which the patient (and therefore the target region) will be moving. Therefore any radiation being guided by these images may suffer from inaccuracies based on the time lag between the position of the target region at the start of imaging, and its position at the time the imaging cycle is finished.

### SUMMARY OF THE INVENTION

It is an aim of the current invention to improve the ability to target a moving region within a patient. To achieve this, more efficient methods of determining the target's position are disclosed.

The invention describes a method whereby instead of repeatedly creating full three-dimensional images of the target region to locate the target, a two-dimensional image is created in a slice through the nominal position of the target that is oriented at an angle substantially orthogonal to the direction of the radiation beam. As the beam moves around the patient, the angle at which the slice is taken is also altered so as to maintain its substantially orthogonal relationship with the radiation beam, the principle being that the position of the target can be visualised in these slices and the properties of the beam adjusted accordingly. This adjustment will typically be done using a multi-leaf collimator (MLC). It can be seen that the position of the target will only be known in two dimensions, due to the single slice, but it is also true that the adjustment of the radiation beam is also only in two dimensions. The orientation of these two sets of dimensions can therefore be continuously matched as the gantry rotates. Furthermore the thickness of the single slice may be adjusted to optimise the signal to noise ratio and also the amount of the target that is included in the slice image. This can be used to optimise the tracking performance.

Slices can be acquired sequentially in two or three orthogonal planes. This allows the system to automatically detect the target in these planes, enhancing the information required to guide the treatment beam. These slices can be through the nominal position of the target. Their orientation may be linked to the direction of the radiation beam or could be fixed.

Thus, according to one embodiment of the present invention, there is provided a radiotherapy apparatus as defined in appended independent claim 1.

In another aspect of the invention, there is provided a radiotherapy apparatus as defined in appended independent claim 5.

Radiotherapy apparatuses according to the preamble of claims 1 and 5 are known from US 2007/0076846 A1 and WO 2005/081842 A2.

The advantage of all of these apparatuses is that because they are not reproducing a full three-dimensional image of the target region, the time taken to produce the images is reduced, thus reducing the delay between any target motion and the corresponding adjustment of the beam position, increasing the accuracy with which the beam can be aimed.

### BRIEF DESCRIPTION OF THE DRAWINGS

An embodiment of the present invention will now be described by way of example, with reference to the accompanying figures in which;
Figure 1 shows a radiotherapy system according to embodiments of the present invention;
Figure 2 is a schematic diagram of aspects of the radiotherapy system according to embodiments of the present invention;
Figure 3 shows a view along the central axis;
Figure 4 shows a beam's eye view of the target region and the imaging planes according to embodiments of the present invention;
Figure 5 shows a view of the target region and the imaging lines according to other embodiments of the present invention; and
Figure 6 shows the variation in location of the imaging lines.

### DETAILED DESCRIPTION OF THE EMBODIMENTS

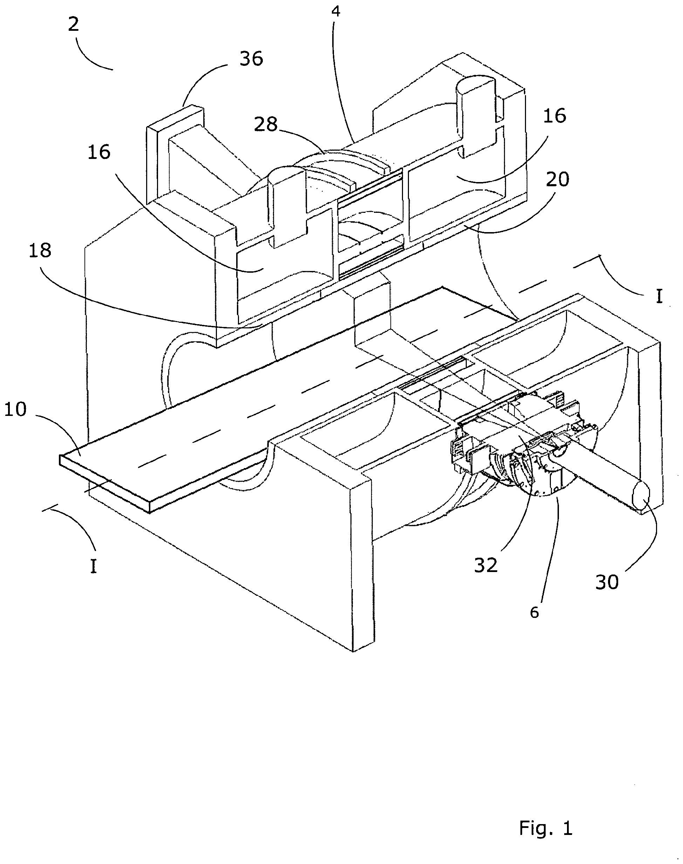

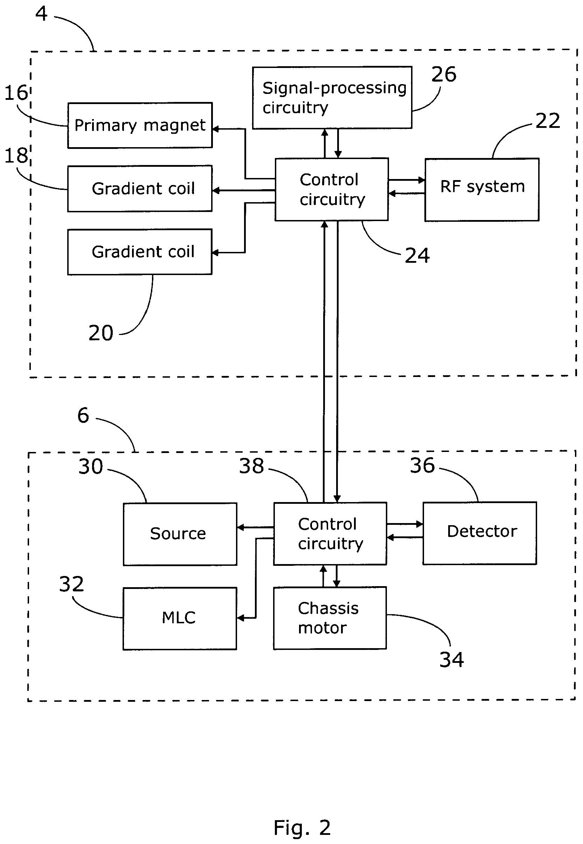

Figure 1 shows a system 2 according to embodiments of the present invention, comprising a radiotherapy apparatus 6 and a magnetic resonance imaging (MRI) apparatus 4. The radiotherapy apparatus 6 and MRI apparatus 4 are shown schematically in Figure 2.

The system includes a couch 10, for supporting a patient in the apparatus. The couch 10 is movable along a horizontal, translation axis (labelled "I"), such that a patient resting on the couch is moved into the radiotherapy and MRI apparatus. In one embodiment, the couch 10 is rotatable around a central vertical axis of rotation, transverse to the translation axis, although this is not illustrated. The couch 10 may form a cantilever section that projects away from a,support structure (not illustrated). In one embodiment, the couch 10 is moved along the translation axis relative to the support structure in order to form the cantilever section, i.e. the cantilever section increases in length as the couch is moved and the lift remains stationary. In another embodiment, both the support structure and the couch 10 move along the translation axis, such that the cantilever section remains substantially constant in length, as described in our US patent application 11/827320 filed on 11 July 2007.

As mentioned above, the system 2 also comprises an MRI apparatus 4, for producing near real-time imaging of a patient positioned on the couch 10. The MRI apparatus includes a primary magnet 16 which acts to generate the so-called "primary" magnetic field for magnetic resonance imaging. That is, the magnetic field lines generated by operation of the magnet 16 run substantially parallel to the central translation axis I. The primary magnet 16 consists of one or more coils with an axis that runs parallel to the translation axis I. The one or more coils may be a single coil or a plurality of coaxial coils of different diameter. In one embodiment (illustrated), the one or more coils in the primary magnet 16 are spaced such that a central window of the magnet 16 is free of coils. In other embodiments, the coils in the magnet 16 may simply be thin enough that they are substantially transparent to radiation of the wavelength generated by the radiotherapy apparatus. The magnet 16 may further comprise one or more active shielding coils, which generates a magnetic field outside the magnet 16 of approximately equal magnitude and opposite polarity to the external primary magnetic field. The more sensitive parts of the system 2, such as the accelerator, are positioned in this region outside the magnet 16 where the magnetic field is cancelled, at least to a first order. The MRI apparatus 4 further comprises two gradient coils 18, 20, which generate the so-called "gradient" magnetic field that is superposed on the primary magnetic field. These coils 18, 20 generate a gradient in the resultant magnetic field that allows spatial encoding of the protons so that their position can be determined, for example the gradient coils 18, 20 can be controlled such that the imaging data obtained has a particular orientation. The gradient coils 18, 20 are positioned around a common central axis with the primary magnet 16, and are displaced from one another along that central axis. This displacement creates a gap, or window, between the two coils 18, 20. In an embodiment where the primary magnet 16 also comprises a central window between coils, the two windows are aligned with one another.

An RF system 22 causes the protons to alter their alignment relative to the magnetic field. When the RF electromagnetic field is turned off the protons return to the original magnetization alignment. These alignment changes create a signal which can be detected by scanning. The RF system 22 may include a single coil that both transmits the radio signals and receives the reflected signals, dedicated transmitting and receiving coils, or multi-element phased array coils, for example. Control circuitry 24 controls the operation of the various coils 16, 18, 20 and the RF system 22, and signal-processing circuitry 26 receives the output of the RF system, generating therefrom images of the patient supported by the couch 10.

As mentioned above, the system 2 further comprises a radiotherapy apparatus 6 which delivers doses of radiation to a patient supported by the couch 10. The majority of the radiotherapy apparatus 6, including at least a source of radiation 30 (e.g. an x-ray source and a linear accelerator) and a multi-leaf collimator (MLC) 32, is mounted on a chassis 28. The chassis 28 is continuously rotatable around the couch 10 when it is inserted into the treatment area, powered by one or more chassis motors 34. In the illustrated embodiment, a radiation detector 36 is also mounted on the chassis 28 opposite the radiation source 30 and with the rotational axis of the chassis positioned between them. The radiotherapy apparatus 6 further comprises control circuitry 38, which may be integrated within the system 2 shown in Figure 1 or remote from it, and controls the radiation source 30, the MLC 32 and the chassis motor 34.

The radiation source 30 is positioned to emit a beam of radiation through the window defined by the two gradient coils 18, 20, and also through the window defined in the primary magnet 16. The radiation beam may be a cone beam or a fan beam, for example.

In other embodiments, the radiotherapy apparatus 6 may comprise more than one source and more than one respective multi-leaf collimator.

In operation, a patient is placed on the couch 10 and the couch is inserted into the treatment area defined by the magnetic coils 16, 18 and the chassis 28. The control circuitry 38 controls the radiation source 30, the MLC 32 and the chassis motor to deliver radiation to the patient through the window between the coils 16, 18. The chassis motor 34 is controlled such that the chassis 28 rotates about the patient, meaning the radiation can be delivered from different directions. The MLC 32 has a plurality of elongate leaves oriented orthogonal to the beam axis; an example is illustrated and described in EP0314214 A2. The leaves of the MLC 32 are controlled to take different positions blocking or allowing through some or all of the radiation beam, thereby altering the shape of the beam as it will reach the patient. Simultaneously with rotation of the chassis 28 about the patient, the couch 10 may be moved along a translation axis into or out of the treatment area (i.e. parallel to the axis of rotation of the chassis). With this simultaneous motion a helical radiation delivery pattern is achieved, known to produce high quality dose distributions.

The MRI apparatus 4, and specifically the signal-processing circuitry 26, delivers real-time (or in practice near real-time) imaging data of the patient to the control circuitry 38. This information allows the control circuitry to adapt the operation of the MLC 32, for example, such that the radiation delivered to the patient accurately tracks the motion of the target region, for example due to breathing.

As mentioned above, conventionally, an MRI apparatus would be used to obtain a full three-dimensional image of the patient. However, this can take a relatively long time, increasing the delay between acquisition of the imaging data, and provision of control signals to the multi-leaf collimator. According to embodiments of the present invention, the MRI apparatus 4 is configured to obtain imaging data comprising two-dimensional slices or one-dimensional line profiles through the target region of a patient, as described in detail below. Such imaging data may then be provided to the control circuitry 38 to allow the radiation beam to be shaped and directed to the target region as appropriate.

### Embodiment 1: Slice image rotating with the beam

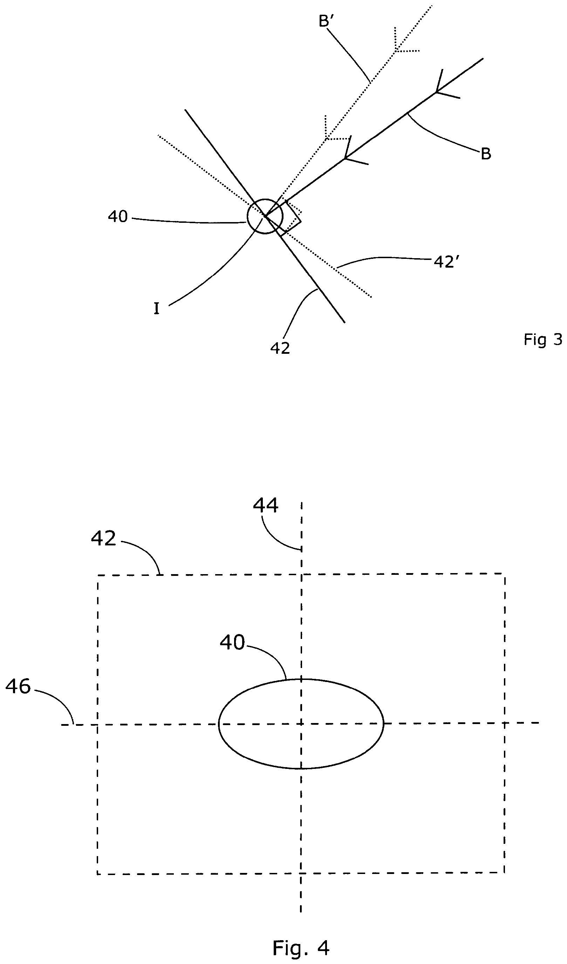

Figure 3 shows a view along the central axis I, and shows the axis B of the beam at an instantaneous orientation relative to the central axis I. Figure 4 shows a beam's eye view of a target area 40 in a patient (e.g. a tumour), and the imaging planes according to embodiments of the invention.

The imaging data obtained by the MRI apparatus 4 comprises at least a two-dimensional slice image of a plane 42 through the target region 40, oriented transverse (i.e. substantially orthogonally) to the axis of the beam B (into the paper as illustrated in figure 4). The imaging data may comprise a plurality of slice images oriented in this direction or, in one embodiment, only a single two-dimensional slice image oriented in this direction. This plane 42 allows the position of the target 40 to be visualized and the position or shape of the beam adjusted accordingly by appropriate positioning of the leaves of the MLC 32. It can be seen that in this embodiment the position of the target will only be known in two dimensions, due to the single slice, but it is also true that the adjustment of the radiation beam position (i.e. by the leaves of the collimator 32) is also only in those same two dimensions. In this way, an image of the target region 40 can be obtained rapidly, and supplied to the collimator 32 for appropriate shaping of the radiation beam before the target moves a significant distance.

According to further embodiments, however, the imaging data obtained by the MRI 4 further comprises one or two two-dimensional slice images of planes oriented orthogonally to the first plane 42. For example, Figure 4 shows a second plane 44 that is orthogonal to the first plane 42, and a third plane 46 that is orthogonal to both first and second planes 42, 44. These three planes may be used to more accurately define the position and shape of the target region 40. All three slice images may still be obtained in a relatively short period of time compared to a full three-dimensional image.

As the beam rotates around the patient, for example to a new instantaneous orientation B' shown in dotted lines in figure 3, the angle at which the slice 42' is taken is also altered so as to maintain its substantially orthogonal relationship with the radiation beam B'. The angles of the other two planes 44, 46 may also be altered so as to maintain their substantially orthogonal relationship with the first plane 42.

### Embodiment 2: Fixed imaging planes

In an alternative aspect not belonging to the invention, the angle at which the two-dimensional slice image is taken may not maintain a substantially orthogonal relationship with the radiation beam as the beam rotates around the patient, but rather be in a substantially fixed orientation relative to the patient. In this aspect, only a single slice image is obtained in any one direction, i.e. slice image 42 is the only image oriented in that particular direction. Further slice images may be obtained in further directions, however. For example, mutually perpendicular slice images 44 and 46 may be obtained, provided these are the only ones oriented in their respective directions.

Thus, the imaging planes are the planes 42, 44, 46 defined above, but are fixed in their orientation relative to the patient and do not rotate to maintain a fixed orientation relative to the beam B.

This aspect has the advantage that imaging data can be obtained even more rapidly, and so the delay between any target motion and the corresponding adjustment of the beam position is reduced still further. Less computational complexity is involved in obtaining slice images with fixed orientations.

The thicknesses of any of the slices discussed above may be adjusted by varying the strength of the gradient field to optimise the signal to noise ratio and also the amount of the target that is included in the particular slice image. This can be used to optimise the tracking performance.

### Embodiment 3: One-Dimensional Samples

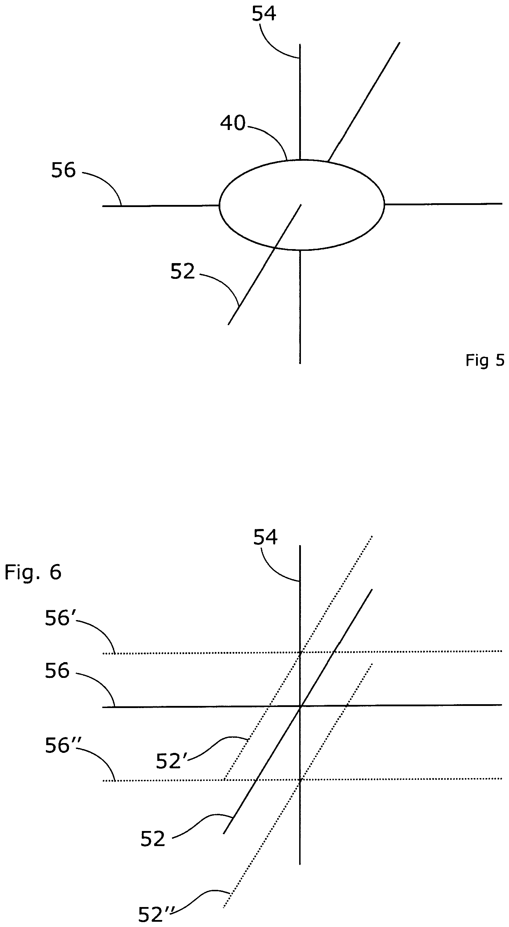

In a further alternative aspect of the invention, the imaging data comprises two or more non-parallel one-dimensional line profiles taken through the target region, such that the profiles contain boundary points between anatomical features (e.g. healthy and cancerous tissue). Figure 5 shows imaging data according to this aspect.

The imaging data obtained by the MRI apparatus 4 in this aspect comprises at least a first one-dimensional line profile 52 through the target region 40 and a second one-dimensional line profile through the target region in a direction non-parallel to the first. In Figure 5, three one-dimensional profiles 52, 54, 56 are illustrated, each perpendicular to the others. However, this aspect of the invention is not limited to orthogonal one-dimensional profiles.

At least one of the line profiles may be oriented substantially orthogonally to the axis of the radiation beam, with the orientation of the line profiles rotating as the radiation beam rotates around the patient to maintain that orthogonal relationship. Alternatively, the orientations of all three line samples may be fixed.

In a further embodiment, the location of the profiles may change as the target region 40 moves. This is shown in figure 6; the profile 54 is fixed in orientation and location, whereas the profiles 52, 56 are fixed in orientation but variable in position. As movement of the target region is detected in an upward direction (as illustrated in figure 6) along the orientation of profile 54, the spatial positions of profiles 52 and 56 are adjusted correspondingly upwardly to 52', 56' as shown. Likewise, as movement of the target region is detected in an downward direction (as illustrated in figure 6) along the orientation of profile 54, the spatial positions of profiles 52 and 56 are adjusted correspondingly downwardly to 52", 56".

In this embodiment, as shown in the arrangement in Figure 6, profile 54 may be termed the "principal axis" and is fixed in one particular location and one particular orientation through the target region 40. For example, the fixed direction may be the predominant direction of motion of the target region, such as up and down a transverse axis of the patient as a result of breathing; in that case the principal axis is chosen parallel to the transverse axis of the patient. However, any direction of motion may be chosen in practice.

The boundary points revealed by such a line profile 54 therefore show the movement of the target region along the principal axis. The other line profiles 52, 56 are chosen in directions that are non-parallel to the first line profile 54 and to each other (e.g. perpendicular). The orientations of the line profiles 52, 56 relative to the first line profile 54 do not change. However, the positions of the line profiles 52, 56 are not fixed and move with the movement of the target region 40 along the principle axis. Thus the first line profile 54 measures the motion of the target region in a particular direction, and the two other line profiles 52, 56 are adjusted to track with that motion.

The boundary points can be combined with knowledge of the shape of the target region (such as, for example, obtained in earlier scans of the target region). This will then reveal, to a sufficient approximation, the location of the target area.

Thus, the target region may be imaged very simply and efficiently in three dimensions using just three one-dimensional line profiles. Such a method achieves excellent temporal resolution, increasing the accuracy with which the radiation beam may be directed.

### Summary

A number of radiotherapy systems are therefore disclosed, each having an imaging apparatus to track movement of the target region and so guide the therapeutic radiation beam during treatment. Various systems are disclosed in which the imaging data obtained by the imaging apparatus is simplified, to reduce the data acquisition time and so improve the temporal resolution of the imaging system and consequently the accuracy of the radiation treatment.

It will of course be understood that many variations may be made to the above-described embodiments without departing from the scope of the present invention, which is defined by the appended claims.

## Claims

1. A radiotherapy apparatus (2), comprising:
a source of radiation (30) for generating a therapeutic beam towards a target region of a patient along a beam axis;
collimation apparatus (32), configured to act on the beam in a plane transverse to the beam axis;
magnetic resonance imaging (MRI) apparatus (4), configured to obtain imaging data of the target region; and
control apparatus (38), configured to receive the imaging data from the MRI apparatus and control the collimation apparatus in dependence thereon,
wherein the imaging data comprises at least first and second non-parallel one-dimensional line profiles (52, 54, 56), the first and second line profiles extending through the target region, and indicating boundary points of the target region in two non-parallel directions, and **characterised in that** the imaging data does not comprise a two-dimensional image or a three-dimensional image of the target region.

2. A radiotherapy apparatus as claimed in claim 1, wherein said first line profile is oriented in a direction transverse to the beam axis.

3. A radiotherapy apparatus as claimed in claim 1, wherein said first line profile is fixed in a direction of motion of the target region.

4. A radiotherapy apparatus as claimed in claim 3, wherein said second line profile is translated in said direction of motion to match movement of the target region in said direction of motion.

5. A radiotherapy apparatus (2), comprising :
a source of radiation (30) for generating a therapeutic beam towards a target region of a patient along a beam axis whose orientation is variable;
collimation apparatus (32), configured to act on the beam in a plane transverse to the beam axis;
magnetic resonance imaging (MRI) apparatus (4), configured to obtain imaging data of the target region, wherein the imaging data comprises at least a first two-dimensional slice image including the target region and being oriented orthogonally to the beam axis, and a control apparatus (38) configured to receive the imaging data from the MRI apparatus and control the collimation apparatus in dependence thereon, **characterised in that** the MRI apparatus is configured to vary an orientation of the first two-dimensional slice image so as to maintain an orthogonal relationship with the beam axis as the orientation of the beam axis varies, and wherein the imaging data does not comprise a three-dimensional image of the target region; and control apparatus, configured to receive the imaging data from the MRI apparatus and control the collimation apparatus in dependence thereon.

6. A radiotherapy apparatus as claimed in claim 5, wherein the imaging data comprises a single two-dimensional slice image oriented in a direction orthogonal to the beam.

7. A radiotherapy apparatus as claimed in claim 5 or 6, wherein the imaging data further comprises a second two-dimensional slice image, the second two-dimensional slice image including the target region and being oriented transverse to the first two-dimensional slice image.

8. A radiotherapy apparatus as claimed in claim 7, wherein the imaging data further comprises a third two-dimensional slice image, the third two-dimensional slice image including the target region and being oriented transverse to the first and second two-dimensional slice images.

## Patentansprüche

1. Strahlentherapievorrichtung (2), umfassend:
eine Strahlungsquelle (30) zum Erzeugen eines therapeutischen Strahls in Richtung auf einen Zielbereich eines Patienten entlang einer Strahlachse;
eine Kollimationsvorrichtung (32), die konfiguriert ist, auf den Strahl in einer Ebene quer zur Strahlachse zu wirken;
eine Magnetresonanztomographie (MRT) -Vorrichtung (4), die konfiguriert ist, Abbildungsdaten des Zielbereichs zu erhalten; und
eine Steuervorrichtung (38), die konfiguriert ist, die Abbildungsdaten von der MRT-Vorrichtung zu empfangen und die Kollimationsvorrichtung in Abhängigkeit davon zu steuern,
wobei die Abbildungsdaten mindestens erste und zweite nicht parallele eindimensionale Linienprofile (52, 54, 56) umfassen, wobei sich das erste und das zweite Linienprofil durch den Zielbereich erstrecken und Grenzpunkte des Zielbereichs in zwei nicht parallelen Richtungen anzeigen, und **dadurch gekennzeichnet, dass**
die Abbildungsdaten kein zweidimensionales Bild oder dreidimensionales Bild des Zielbereichs umfassen.

2. Strahlentherapievorrichtung nach Anspruch 1, wobei das erste Linienprofil in einer Richtung quer zur Strahlachse ausgerichtet ist.

3. Strahlentherapievorrichtung nach Anspruch 1, wobei das erste Linienprofil in einer Bewegungsrichtung des Zielbereichs fixiert ist.

4. Strahlentherapievorrichtung nach Anspruch 3, wobei das zweite Linienprofil in die Bewegungsrichtung translatiert wird, um der Bewegung des Zielbereichs in der Bewegungsrichtung zu entsprechen.

5. Strahlentherapievorrichtung (2), umfassend:
eine Strahlungsquelle (30) zum Erzeugen eines therapeutischen Strahls in Richtung auf einen Zielbereich eines Patienten entlang einer Strahlachse, deren Ausrichtung variabel ist;
eine Kollimationsvorrichtung (32), die konfiguriert ist, auf den Strahl in einer Ebene quer zur Strahlachse zu wirken;
Magnetresonanztomographie (MRT) -Vorrichtung (4), die konfiguriert ist, Abbildungsdaten des Zielbereichs zu erhalten, wobei die Abbildungsdaten mindestens ein erstes zweidimensionales Schnittbild umfassen, das den Zielbereich aufweist, und orthogonal zur Strahlachse ausgerichtet ist, und
eine Steuervorrichtung (38), die konfiguriert ist, die Abbildungsdaten von der MRT-Vorrichtung zu empfangen und die Kollimationsvorrichtung in Abhängigkeit davon zu steuern, **gekennzeichnet dadurch, dass**
die MRT-Vorrichtung konfiguriert ist, eine Ausrichtung des ersten zweidimensionalen Schnittbildes zu variieren, um eine orthogonale Beziehung zur Strahlachse aufrechtzuerhalten, wenn die Ausrichtung der Strahlachse variiert, und wobei die Abbildungsdaten kein dreidimensionales Bild des Zielbereichs umfassen; und
eine Steuervorrichtung, die konfiguriert ist, die Abbildungsdaten von der MRT-Vorrichtung zu empfangen und die Kollimationsvorrichtung in Abhängigkeit davon zu steuern.

6. Strahlentherapievorrichtung nach Anspruch 5, wobei die Abbildungsdaten ein einzelnes zweidimensionales Schnittbild umfassen, das in einer Richtung orthogonal zum Strahl ausgerichtet ist.

7. Strahlentherapievorrichtung nach Anspruch 5 oder 6, wobei die Abbildungsdaten ferner ein zweites zweidimensionales Schnittbild umfassen, wobei das zweite zweidimensionale Schnittbild den Zielbereich aufweist und quer zum ersten zweidimensionalen Schnittbild ausgerichtet ist.

8. Strahlentherapievorrichtung nach Anspruch 7, wobei die Abbildungsdaten ferner ein drittes zweidimensionales Schnittbild umfassen, wobei das dritte zweidimensionale Schnittbild den Zielbereich aufweist und quer zu dem ersten und zweiten zweidimensionalen Schnittbild ausgerichtet ist.

## Revendications

1. Appareil de radiothérapie (2), comprenant :
une source de radiation (30) pour générer un faisceau thérapeutique vers une zone cible d'un patient le long d'un axe de faisceau ;
un appareil de collimation (32) conçu pour agir sur le faisceau dans un plan transversal à l'axe du faisceau ;
un appareil d'imagerie à résonance magnétique (IRM) (4) conçu pour obtenir des données d'imagerie de la zone cible ; et
un appareil de commande (38) conçu pour recevoir les données d'imagerie provenant de l'appareil d'IRM et commander l'appareil de collimation en fonction de celles-ci,
les données d'imagerie comprenant au moins des premier et second profils de ligne unidirectionnelle non parallèle (52, 54, 56), les premier et second profils de ligne unidirectionnelle s'étendant à travers la zone cible, et indiquant des points limites de la zone cible dans deux directions non parallèles, et **caractérisé en ce que**
les données d'imagerie ne comprennent pas une image bidimensionnelle ou une image tridimensionnelle de la zone cible.

2. Appareil de radiothérapie selon la revendication 1, dans lequel ledit profil de ligne est orienté dans une direction transversale à l'axe du faisceau.

3. Appareil de radiothérapie selon la revendication 1, dans lequel ledit premier profil de ligne est fixe dans une direction de mouvement de la zone cible.

4. Appareil de radiothérapie selon la revendication 3, dans lequel ledit second profil de ligne est transféré dans ladite direction de mouvement pour correspondre au mouvement de la zone cible dans ladite direction de mouvement.

5. Appareil de radiothérapie (2), comprenant :
une source de radiation (30) pour générer un faisceau thérapeutique vers une zone cible d'un patient le long d'un axe de faisceau dont l'orientation est variable ;
un appareil de collimation (32) conçu pour agir sur le faisceau dans un plan transversal à l'axe du faisceau ;
un appareil d'imagerie à résonance magnétique (IRM) (4) conçu pour obtenir des données d'imagerie de la zone cible, les données d'imagerie comprenant au moins une première image de vue bidimensionnelle incluant la zone cible et qui est orientée orthogonalement par rapport à l'axe du faisceau, et
un appareil de commande (38) conçu pour recevoir les données d'imagerie provenant de l'appareil d'IRM et commander l'appareil de collimation en fonction de celles-ci, **caractérisé en ce que**
l'appareil d'IRM est conçu pour varier une orientation de la première image en vue bidimensionnelle de manière à maintenir une relation orthogonale avec l'axe de faisceau lorsque l'orientation de l'axe de faisceau varie, et les données d'imagerie ne comprennent pas une image tridimensionnelle de la zone cible ; et
un appareil de commande conçu pour recevoir des données d'imagerie provenant de l'appareil d'IRM et commander l'appareil de collimation en fonction de celles-ci.

6. Appareil de radiothérapie selon la revendication 5, dans lequel les données d'imagerie comprennent une image en vue bidimensionnelle unique orientée dans une direction orthogonale au faisceau.

7. Appareil de radiothérapie selon la revendication 5 ou 6, dans lequel les données d'imagerie comprennent en outre une deuxième image en vue bidimensionnelle, la deuxième image en vue bidimensionnelle incluant la zone cible et étant orientée transversalement à la première image en vue bidimensionnelle.

8. Appareil de radiothérapie selon la revendication 7, dans lequel les données d'imagerie comprennent en outre une troisième image en vue bidimensionnelle, la troisième image en vue bidimensionnelle incluant la zone cible et étant orientée transversalement aux première et deuxième image en vue bidimensionnelle.
